# EUROPEAN PATENT SPECIFICATION

(11) **EP 3 162 898 B1**
(45) Date of publication and mention of the grant of the patent: **12.12.2018**
(21) Application number: 16195937.4
(22) Date of filing: 27.10.2016
(51) Int. Cl.: C12P 5/02, C12R 1/145

(54) **PROCESS FOR THE PRODUCTION OF BIOGAS FROM A SOLID DIGESTATE**
VERFAHREN ZUR HERSTELLUNG VON BIOGAS AUS EINEM FESTEN GÄRREST
PROCÉDÉ DE PRODUCTION DE BIOGAZ À PARTIR D'UN DIGESTAT SOLIDE

(30) Priority: 27.10.2015 IT UB20154915
(43) Date of publication of application: 03.05.2017
(73) Proprietor: TIRSI S.r.l., 27032 Ferrera Erbognone (IT)
(72) Inventor: SCEVOLA, Mario Ercole, 20146 MILANO (IT); DELLE CANNE, Marco Gioacchino, 20124 MILANO (IT); SPAGNOL, Manuela, 27029 VIGEVANO (IT)
(74) Representative: Coppo, Alessandro

(56) References cited:
- WO-A2-2011/014894
- DATABASE EPODOC [Online] EUROPEAN PATENT OFFICE; 5 March 2002 (2002-03-05), "GARBAGE BIOGASIFICATION DEVICE", XP002759647, Database accession no. JP-2000264072-A -& JP 2002 066518 A (TOSHIBA CORP) 5 March 2002 (2002-03-05)
- LU, Y. ET AL.: "Characteristics of hydrogen and methane production from cornstalks by an augmented two- or three-stage anaerobic fermentation process", BIORESOURCE TECHNOLOGY, vol. 100, no. 12, 23 February 2009 (2009-02-23), pages 2889-2895, XP026027136,
- WEIMER, P.J. & ZEIKUS, J.G.: "Fermentation of Cellulose and Cellobiose by Clostridium thermocellum in the Absence and Presence of Methanobacterium thermoautotrophicum", APPLIED AND ENVIRONMENTAL MICROBIOLOGY, vol. 33, no. 2, February 1977 (1977-02), pages 289-297, XP001315239,

## Description

### FIELD OF THE INVENTION

The present invention relates to a process for the production of biogas from the solid fraction of a waste digestate.

The present invention originates in the field of biogas production.

More specifically, the invention relates to a process for the treatment and fermentation of the solid fraction of digestate obtained by anaerobic digestion of organic substances contained in biomasses.

The invention further relates to the use of selected bacteria of the species *Clostridium thermocellum* for the treatment of solid digestate and for the production of biogas.

### BACKGROUND OF THE ART

In recent decades, the growing need of disposing waste material, effluents of breeding and plant biomasses, along with the need of energy sources alternative to oil, has given momentum to development of technologies for the production of biogas.

As is well known, biogas is obtained as the product of processes of anaerobic digestion of organic substances contained in a biomass.

In more detail, in anaerobic digestion processes, starting biomasses contain organic material that is degraded by mesophilic or thermophilic microorganisms under anaerobic conditions within suitable containers called digesters or biodigesters. The anaerobic digestion process produces biogas and digestate as main products. The first one, biogas, is essentially formed by a mixture of methane and carbon dioxide, the second one, the digestate, comprises two components or fractions, one of which is solid while the other is liquid.

The liquid or clarified fraction is about 85-90% of the volume of the digestate and generally has a dry matter content in the range of 1.5 to 8%. Soluble compounds, such as nitrogen in the form of ammonia, are collected in this fraction and can account for up to 70-90% by weight of the total quantity of nitrogen present in the digestate. The high content of ammonia nitrogen makes it a product suitable for agronomic use, in particular as fertilizer, for example for application in fertigation.

The solid or generally shovelable fraction represents about 10-15% of the weight of the digestate as such, and it is characterized by a dry matter content typically exceeding 20% by weight. This fraction is currently used in the agronomic field as a fertilizer able to slowly release the nutrients.

The publication JP 2002 066518A discloses a process for biogas production comprising the step of:
a) contacting a solid fraction of a digestate in a biodigester with hydrolytic bacteria, causing an anaerobic digestion with lysis and formation of a degraded digestate containing free sugars to be recycled to a gas production plant,
b) addition of the degraded digestate obtained in step a) to a biomass to be subjected to anaerobic digestion with formation of biogas, wherein said solid fraction of the digestate is obtained by mechanical separation of an aqueous suspension obtained as a product from biogas plants, wherein the fermentation takes place in a bioreactor.

Lu, Y. et al. "Characteristics of hydrogen and methane production from cornstalks by an augmented two- or three stage anaerobic fermentation process" in Bioresource Technology, vol. 100, no. 12, 23.03.2009 pages 2889-2895 discloses the use of cellulolytic bacteria of the species Clostridium thermocellum ATCC 1549 for the anaerobic digestion of a solid plant substrate based on cellulosic fibers with formation of simple sugars, thereby enhancing the lysis of cellulosic fibers in the hydrolysis step, with the formation of increased yields of free sugars for the biogas production process, and the use of cellulolytic bacteria of the species clostridium thermocellum ATCC1549 in biogas production.

The increasing demand for technology capable of disposing of or transforming waste materials, waste water and waste products from agricultural production in potential sources of energy is one factor that has contributed to the development of technologies for the production of biogas.

The production of biogas is further promoted by the fact that the electrical energy produced from the waste materials is considered a form of green energy, which is currently much in demand by the market.

Furthermore, biogas obtained by digestion of waste materials is particularly appreciated, since it is considered a source of energy with low environmental impact.

Currently, there is a need to improve the efficiency of anaerobic digestion processes in order to increase the yields of biogas production.

A general object of the present invention is therefore to improve the yields of biogas production by anaerobic digestion process of biomasses.

A further object of the invention is to provide a biomass anaerobic digestion process in which the production of biogas is increased with respect to a traditional process, with the same quantity of starting biomass.

### SUMMARY OF THE INVENTION

According to a general aspect, the inventors have found the possibility of increasing the production yield of biogas obtained from traditional processes of biomass anaerobic digestion by a treatment of plant components contained in the solid fraction of the digestate.

The invention process thus increases the efficiency of biogas production processes by providing a further specific anaerobic digestion step on the solid fraction of the digestate, in particular of vegetable origin. This treatment step produces further biogas and more degraded digestate that can still be used, for example, in the field of agronomy.

According to a general aspect, a process for biogas production is disclosed, including a step in which the solid fraction of the digestate is brought into contact with cellulolytic bacteria of the *Clostridium thermocellum* species, thus causing a lysis of cellulosic fibers, with formation of free sugars to be used for the production of biogas.

Microorganisms suitable for use within this disclosure are cellulolytic bacterial strains belonging to the *Clostridium* genus, *Clostridium thermocellum* species which typically can be isolated and selected from green compost, deteriorated wood of trees such as poplars, swamp waters, waters from hemp maceration. Within the present disclosure, the Applicant has also selected a number of specific bacterial strains belonging to the *Clostridium* genus provided with remarkably elevated cellulolytic activity. These selected cellulolytic strains are specifically applied to the invention process thus causing anaerobic digestion of digestate, in particular of plant origin, with high yields.

According to one aspect, the invention therefore provides the use of a mixture of strains of cellulolytic *Clostridium thermocellum*
having the following access numbers:
DSM 32298 identification reference SCEVOLA_1
DSM 32299 identification reference SCEVOLA_2
DSM 32310 identification reference SCEVOLA_3, in biogas production.

The access number has been assigned by the International Deposit Authority DSMZ, at Leibniz Institute DSMZ- German Collection of Microorganisms and Cell Cultures, Inhoffenstr.7 B, D-38124 Braunschweig. These strains were deposited on May 20, 2016 by the applicant Tirsi srl, Via Cascina Gallona, 27032 Ferrera Erbognone (PV). Certificates of deposit of microorganisms, made according to the Budapest Treaty, are attached and filed with the present application.

Typically, a mixture of the three previously described bacterial strains, comprising in equal parts the respective cultures in liquid growth medium, is used in the process of the invention.

### DESCRIPTION OF THE DIAGRAMS, FIGURE AND SCHEME

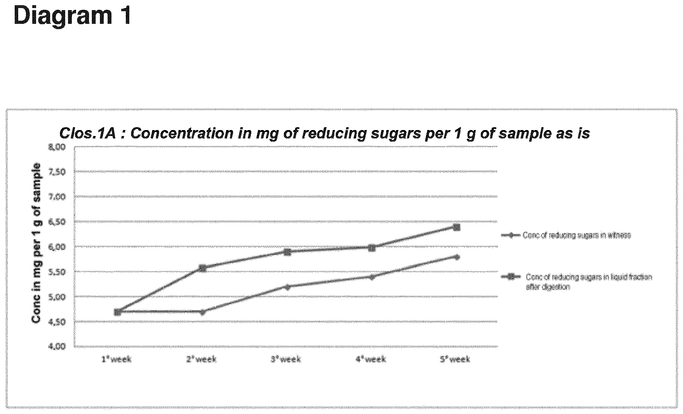

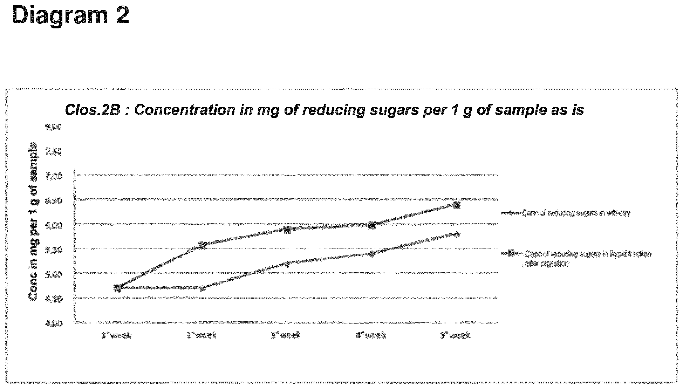

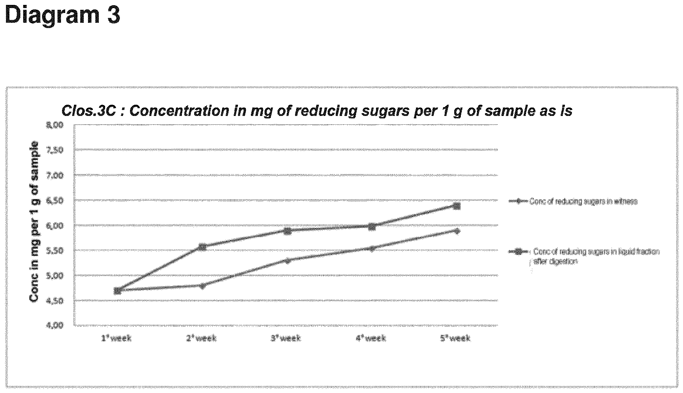

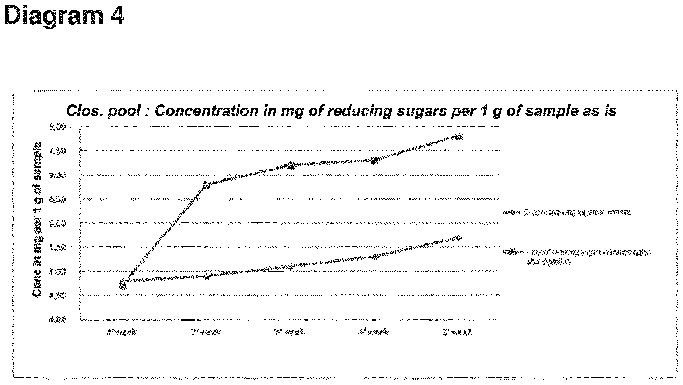

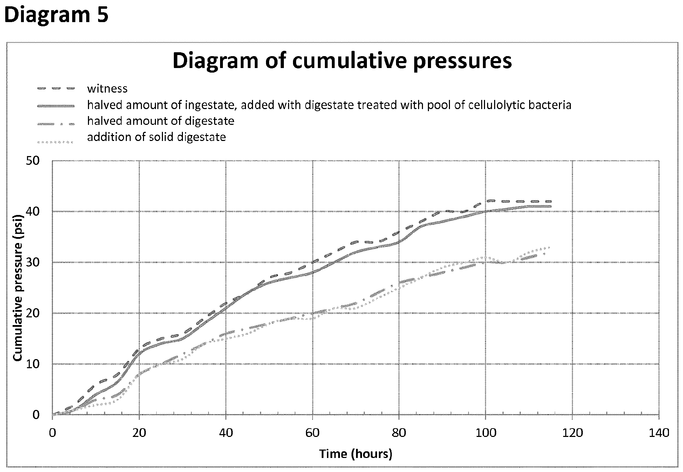

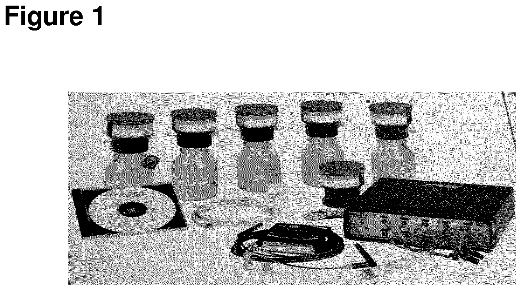

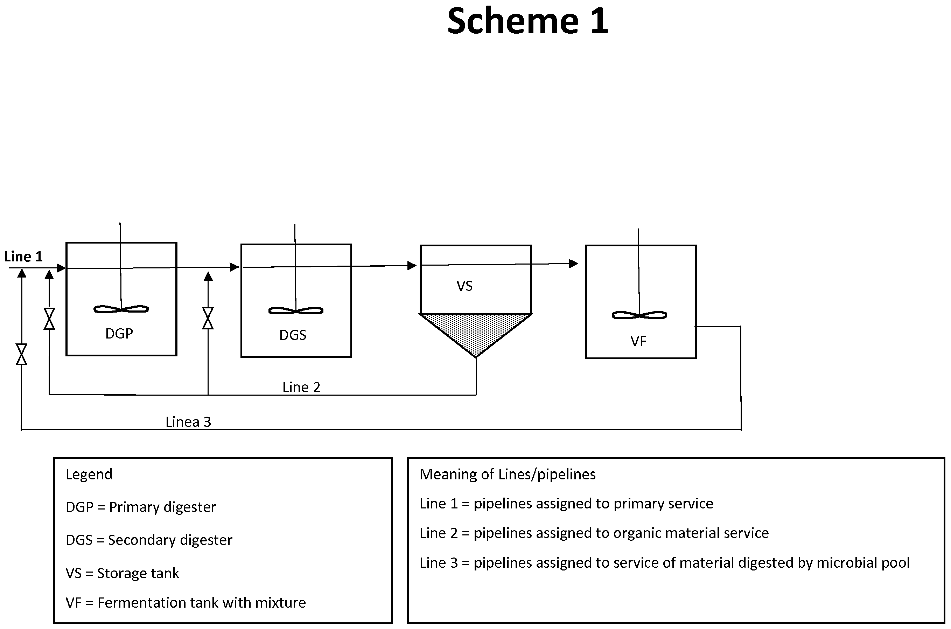

The invention will now be described in detail with reference to the attachments, in which:
Diagram 1 shows the average values of free sugars found as a function of incubation time for the strain *DSM 32298,* as explained in Example 1;
Diagram 2 shows the average values of free sugars found as a function of incubation time for the strain *DSM 32299,* as explained in Example 2;
Diagram 3 shows the average values of free sugars found as a function of incubation time for the strain *DSM 32310,* as explained in Example 3;
Diagram 4 shows the average values of free sugars found as a function of incubation time for a mixture of the three strain *DSM 32298, DSM 32299* and *DSM 32310,* as explained in Example 4;
Diagram 5 shows the biogas pressure values, as explained in Example 5; Figure 1 shows the RFS Ankon Gas Production Wireless System apparatus, used to monitor the pressure of the biogas;
Scheme 1 describes the process for the treatment and recycling of the digestate in biogas production.

### DETAILED DESCRIPTION OF THE INVENTION

In the context of the present disclosure, the inventors have isolated bacterial strains belonging to the *Clostridium* genus provided with a particularly high cellulolytic activity and which are therefore specifically applicable to anaerobic digestion of the solid component of the digestate, in particular of plant origin. A product rich in sugars, that can be recycled in the production of biogas cycle, is obtained by anaerobic digestion of the solid digestate component with the selected cellulolytic strains used in the invention.

In some embodiments, the solid fraction of the digestate is obtained by mechanical separation, by filtration of a waste aqueous suspension in biogas facilities.

In accordance with an aspect, the present invention relates to a process for the treatment and recycling of digestate in the production of biogas, comprising the steps of:
a) Contacting a solid fraction of a digestate, containing cellulose and/or hemicellulose, in a biodigester with a mixture of cellulolytic *Clostridium thermocellum* strains *DSM 32298, DSM 32299* and *DSM 32310* causing an anaerobic digestion with lysis of the cellulose and/or hemicellulose and formation of a degraded digestate containing free sugars to be recycled to a gas production plant,
b) Addition of the degraded digestate obtained in step a) to a biomass to be subjected to anaerobic digestion with formation of biogas.

Advantageously, the addition of the digestate previously treated with cellulolytic bacterial strains in the bioreactor causes a reduction of biomass to be subjected to anaerobic digestion, with the same yield of biogas production.

In the present invention, the word "biogas" means a mixture of gases based on methane (CH₄) and carbon dioxide (CO₂), typically produced by anaerobic digestion of an ingestate in a biogas production plant.

Within the scope of the invention, the words biomass and ingestate mean the starting material of an anaerobic digestion process. Ingestate may comprise one or more of urban and non-urban waste materials, waste of animal farms, waste material from agricultural production and biomasses.

An ingestate suitable for the applications of the invention includes organic residues of vegetable and/or animal origin and in particular containing cellulosic fibers.

Particularly suitable biomass may have different origins, and typically can be waste from agriculture industry, such as shredded corn, triticale, sorghum or other crops, waste or scraps from the food industry, such as waste flour or expired products of plant origin, animal or livestock waste.

Typically, the solid fraction of the digestate contains cellulose and/or hemicellulose, e.g., corn silage.

A physicochemical analytical study of the solid fraction of the digestate, carried out on samples taken under different operating and seasonal conditions in connection with the collection of ingestate, such as corn silage, has shown that this solid fraction has an appreciable content of cellulose and hemicellulose.

In particular, in those cases where the amount of biogas being produced tends to decrease during the process, an increase in the content of non metabolized cellulose and hemicellulose can be observed in the solid digestate, which makes its recycling after treatment even more convenient.

In one embodiment of the process of the present invention, the solid digestate is subjected to the action of the mixture of the thermophilic anaerobic microorganisms having high cellulolytic activity capable of hydrolyzing the cellulosic components still present in the solid fraction of the digestate, i.e. the mixture of the *Clostridium thermocellum* strains DSM 32298, DSM 32299, DSM 32310, with formation of free sugar, mainly glucose, typically at a temperature ranging from 30 °C to 50 °C, preferably from 38 °C to 48 °C.

The addition of the digested suspension previously treated with cellulolytic bacterial strains in the bioreactor for methanogenesis, according to any one of the embodiments described above, therefore containing free sugars, allows a reduction of up to 50% of the amount of ingestate (corn silage) with the same yield of biogas production.

In the process according to the present invention, a mixture of the three strains of bacteria *DSM 32298, DSM 32299* and *DSM 32310* of the *Clostridium thermocellum* species is used. Surprisingly, it has been observed that the mixture of these three strains of microorganisms possesses cellulolytic activity greater than that of each single one, showing higher enzymatic activity as reported in the examples shown below.

The simple sugars solution obtained from the lysis of cellulosic fibers added in the biodigester has been shown to provide a yield in biogas production (methane + 52-56% carbon dioxide 30-35%) equal to that of the witness test containing a double amount of ingestate (corn silage). Further disclosed is the use of a mixture of bacteria strains *DSM 32298, DSM 32299* and *DSM 32310,* of the *Clostridium thermocellum* species, in the treatment of solid digestate based on cellulosic fibers, which recycled in the biodigester of a biogas production plant causes the production of methane with consequent saving of ingestate.

The exemplary embodiments of the present invention provided by way of illustrative examples are reported below.

### EXAMPLES

### Example 1

### Isolation of the strain DSM 32298

The *DSM 32298* strain was isolated in autumn from residues of trunks of poplars deteriorated by successive transplants in solid culture media (agar) and liquid culture media (eg. potato dextrose).

### Hydrolysis test with strain DSM 32298

Hydrolysis of residual cellulosic fibers using a solid digestate representative sample taken in an industrial plant fed with biogas ingestate consisting of green defibrated corn having average content of 32% s.s. and hemicellulose 15% s.s. 100 g of solid digestate suspended in 100 ml of 0.1M phosphate buffer with pH 6.4 and seeded with 100 ml of broth culture of *Clostridium* called *DSM 32298* were put into three airtight glass containers. All containers were insufflated with nitrogen in order to ensure an anaerobic environment. After taking a sample for the determination of possible presence of free sugars, the containers were tightly sealed and placed in a thermostat under shaking at 40 °C for 15 days.

As a control (witness) of test reference as many identical containers were set up in parallel replacing sowing with broth culture of the fibrinolytic activity strain *DSM 32298* with an equal volume of 100 ml of not inoculated culture medium. These control containers were subjected to anaerobic conditions with nitrogen and incubated in an identical manner as those of the test.

At the end of the incubation period, the contents of each container was filtered through a mesh and the determination, by means of Bailey analytical method, of free sugars present in the liquid fractions was carried out.

Diagram 1 shows the average values of free sugars found as a function of the incubation time, which shows that strain *DSM 32298* (*Clos.*1A) degrades 18% of cellulosic fibers less than the mixture of the 3 *Clostridium* strains (see Example 4).

### Example 2

### Isolation of the strain DSM 32299

The *DSM 32299* strain was isolated from samples of maturing green compost using the same technique as in Example 1.

### Hydrolysis test with strain DSM 32299

The cellulolytic activity of the second *Clostridium* strain called *DSM 32299* was tested in a way entirely analogous to that reported in Example 1.

Diagram 2 shows the average values of free sugars found as a function of the incubation time, which shows that *DSM 32299* strain (*Clos.* 2B) degrades 16% of cellulosic fibers less than the mixture of the 3 *Clostridium* strains (see Example 4).

### Example 3

### Isolation of the strain DSM 32310

The *DSM 32310* strain was isolated from marsh water using the same technique as in Example 1.

### Hydrolysis test with strain DSM 32310

Using the *Clostridium* strain called *DSM 32310* (*Clos.* 3C) (Diagram 3), the cellulolytic activity was tested with same modes as those described in Example 1, with the result that strain *DSM 32310* degrades 12% less as compared to the mixture of the 3 strains of *Clostridium* (see Example 4).

### Example 4

By operating in a similar way to what reported in Example 1 but using a mixture in equal parts of the three strains *DSM 32298* - *DSM 32299* - *DSM 32310* in a culture broth as a seed, a content of free sugars was found increased by an average of 15% with respect to that found in the tests with individual bacteria strains (Diagram 4).

### Example 5

Test of biogas production using the free sugar solution obtained in Example 4.

For the tests of methanogenesis the Ankom equipment RFS Wireless Gas Production System (Figure 1) was used, which allows monitoring of the biogas pressure over time (Diagram 5). 100 g of the culture broth coming from Biogas industrial plant were placed in each container, and then the test was prepared as follows:
a) Container No. 1 considered as witness test: 12.5 g of corn silage (corresponding to 100% of supply) and 100 ml of 0.1 M phosphate buffer pH 6.4.
b) Containers No. 2 and No. 3 duplicate biogas production tests: 6 g of silage (corresponding to 50% of supply) with 100 ml of the liquid fraction of the digestate treated with the mixture of cellulolytic microorganisms.
c) Container No. 4 control test: 6 g of silage and 100 ml of 0.1 M phosphate buffer pH 6.4.
d) Container No. 5 control of the treatment: 6 g silage + 6 g solid digestate untreated and 100 ml of 0.1 M phosphate buffer pH 6.4.

Nitrogen was insufflated in each container for 3 minutes in order to create an anaerobic environment. The containers so prepared were incubated at 40 °C under slow orbital agitation (40-70 rpm).

Subsequenlty the quality of the biogas produced was analyzed by Portable Gas Chromatograph (MICRO GC 490) with Molsieve 5A column and Sil 5 CB, to differentiate the main gases produced by methanogenic reaction including methane and carbon dioxide.

The same qualitative composition of methane biogas consisting of 52-56% and 30-35% carbon dioxide was found in the tests. The diagram obtained by Ankom RFS Gas Production Wireless System (Diagram 5) shows the same amount of biogas produced in the test by halving the amount of silage with the addition of the liquid fraction of the digestate, after that it was subjected to the action of the mixture of cellulolytic bacteria.

From the detailed description and from the Examples given above, the advantages achieved by the process of the present invention are evident. In particular, this method has proved surprisingly suitable to obtain a yield in biogas production equal to that of the witness test containing a double amount of ingestate (corn silage).

### EXAMPLE 6

A process for the treatment and recycling of digestate in biogas production and a plant for its realization are illustrated in Scheme 1.

A bioreactor called VF is initially loaded with solid fraction of the digestate containing cellulose and/or hemicellulose. A mixture of cellulolytic bacterial strains *Clostridium sp DSM 32298, DSM 32299* and *DSM 32310* is added producing a fermentation with lysis of the cellulose and/or hemicellulose contained in the digestate and formation of a degraded digestate containing free sugars.

The degraded digestate is recycled through line 3 to a primary digester (DGP) in which the anaerobic degradation occurs. The product obtained from the anaerobic digestion is then transferred to a secondary digester (DGS) in which the anaerobic digestion progresses. The product resulting from the fermentation is then transferred to a storage tank (VS) from which it can then be either transferred to a fermentation tank VF or recycled to the primary digester DGP through line 2.

## Claims

1. A process for biogas production, comprising the steps of:
a) contacting a solid fraction of a digestate containing cellulose and/or hemicellulose in a biodigester with cellulolytic bacteria of the species *Clostridium thermocellum,* causing an anaerobic digestion with lysis of the cellulose and/or hemicellulose and formation of a degraded digestate containing free sugars to be recycled to a gas production plant,
b) addition of the degraded digestate obtained in step a) to a biomass to be subjected to anaerobic digestion with formation of biogas wherein the cellulolytic bacteria are a mixture of strains *DSM* 32298, *DSM* 32299 *DSM* 32310.

2. The process according to claim 1, wherein said solid fraction of the digestate contains cellulose and/or hemicellulose.

3. The process according to anyone of claims 1 or 2, wherein said solid fraction of the digestate is obtained by mechanical separation, preferably by filtration, of an aqueous suspension obtained as a product from biogas plants.

4. The process according to anyone of claims 1 to 3, wherein the fermentation takes place in a bioreactor, preferably in an anaerobic environment at a temperature comprised between 30°C and 50°C.

5. Use of the mixture of strains of cellulolytic *Clostridium thermocellum DSM* 32298, *DSM* 32299, *DSM* 32310 in biogas production.

## Patentansprüche

1. Verfahren zur Herstellung von Biogas, aufweisend folgende Schritte:
a) Kontaktieren eines festen Anteils eines Gärrests, der Zellulose und / oder Hemizellulose enthält, mit zellulolytischen Bakterien der Art *Clostridium thermocellum* in einem Biodigester, zum Herbeiführen einer anaeroben Digestion, mit Lyse der Zellulose und / oder Hemizellulose und Bildung eines degradierten Gärrests, der freien Zucker enthält, der an eine Gaserzeugungsanlage zurückgeführt wird,
b) Hinzufügen des in Schritt a) erhaltenen degradierten Gärrests zu einer Biomasse, die der anaeroben Digestion unter Bildung von Biogas unterworfen wird, wobei die zellulolytischen Bakterien eine Mischung aus den Stämmen DSM 32298, DSM 32299 und DSM 32310 sind.

2. Verfahren gemäß Anspruch 1, wobei der feste Anteil des Gärrests Zellulose und / oder Hemizellulose enthält.

3. Verfahren gemäß einem der Ansprüche 1 oder 2, wobei der feste Anteil des Gärrests durch mechanisches Abscheiden, vorzugsweise durch Filtration, einer wässrigen Suspension erhalten wird, die als Produkt aus Biogasanlagen erhalten wird.

4. Verfahren gemäß einem der Ansprüche 1 bis 3, wobei die Fermentation in einem Bioreaktor stattfindet, vorzugsweise in anaerober Umgebung bei einer Temperatur zwischen 30 °C und 50 °C.

5. Verwendung der Mischung aus Stämmen von zellulolytischem *Clostridium thermocellum* DSM 32298, DSM 32299 und DSM 32310 in der Biogasherstellung.

## Revendications

1. Procédé de production de biogaz, comprenant les étapes de :
a) mise en contact d'une fraction solide d'un digestat contenant de la cellulose et/ou de l'hémicellulose dans un biodigesteur avec des bactéries cellulolytiques de l'espèce *Clostridium thermocellum,* entraînant une digestion anaérobie avec une lyse de la cellulose et/ou de l'hémicellulose et une formation d'un digestat dégradé contenant des sucres libres à recycler vers une installation de production de gaz,
b) addition du digestat dégradé obtenu dans l'étape a) à une biomasse à soumettre à une digestion anaérobie avec formation de biogaz, dans lequel les bactéries cellulolytiques sont un mélange de souches *DSM* 32298, *DSM* 32299, *DSM* 32310.

2. Procédé selon la revendication 1, dans lequel ladite fraction solide du digestat contient de la cellulose et/ou de l'hémicellulose.

3. Procédé selon l'une quelconque des revendications 1 ou 2, dans lequel ladite fraction solide du digestat est obtenue par séparation mécanique, de préférence par filtration, d'une suspension aqueuse obtenue sous forme d'un produit issu d'installations de biogaz.

4. Procédé selon l'une quelconque des revendications 1 à 3, dans lequel la fermentation a lieu dans un bioréacteur, de préférence dans un environnement anaérobie à une température comprise entre 30 °C et 50 °C.

5. Utilisation du mélange de souches de *Clostridium thermocellum* cellulolytiques *DSM* 32298, *DSM* 32299, *DSM* 32310 dans la production de biogaz.
